# EUROPÄISCHE PATENTANMELDUNG

(11) **EP 4 763 043 A1**
(43) Veröffentlichungstag der Anmeldung: **24.06.2026**
(21) Anmeldenummer: 25223585.8
(22) Anmeldetag: 15.12.2025
(51) Int. Cl.: A61B 1/00, A61B 1/06, A61B 1/07, A61B 1/05, A61B 1/12

(54) **ENDOSKOPVORRICHTUNG UND SYSTEM MIT EINER ENDOSKOPVORRICHTUNG**

(30) Priorität: 19.12.2024 DE 102024138868
(71) Anmelder: Karl Storz SE & Co. KG, 78532 Tuttlingen (DE)
(72) Erfinder: SCHWARZ, Peter, 78532 Tuttlingen (DE)

(57) **Zusammenfassung**

Die Erfindung betrifft eine Endoskopvorrichtung (10), umfassend: einen Schaft (20) mit einer Schaftlängsachse (30); einen Betätigungsschaft (40), der parallel zur Schaftlängsachse (30) bewegbar ist; und ein Kameramodul (50), das schwenkbar an dem Schaft (20) gelagert ist und eine stereoskopische Bilderfassungseinheit (60) aufweist, wobei das Kameramodul (50) durch ein Verschieben des Betätigungsschafts (40) relativ zum Kameramodul (50) parallel zu der Schaftlängsachse (30) und/oder durch ein Verschieben des Kameramoduls (50) relativ zum Betätigungsschaft (40) von einer Einführlage in eine Bildaufnahmelage auslenkbar ist, wobei in der Bildaufnahmelage das Kameramodul (50) eine Blickrichtung definiert, die zu der Schaftlängsachse (30) abgewinkelt ist, und wobei in der Bildaufnahmelage die stereoskopische Bilderfassungseinheit (60) zu einer Bildaufrichtung relativ zu dem Betätigungsschaft (40) drehbar gelagert ist.

Weiter betrifft die Erfindung ein System mit einer erfindungsgemäßen Endoskopvorrichtung.

## Beschreibung

Die vorliegende Erfindung betrifft eine Endoskopvorrichtung, insbesondere ein Stereo-Laparoskop, und ein System mit einer Endoskopvorrichtung.

In der minimalinvasiven Chirurgie gehören Endoskope zum Stand der Technik, mittels derer Vergrößerungsdarstellungen eines Untersuchungsgebiets innerhalb eines Körpers eines Patienten erzeugt werden können. Zur besseren Visualisierung werden in Endoskopen stereoskopische Bilderfassungseinheiten eingesetzt, die die Umgebung einem Operateur und/oder einem weiteren Benutzer, in 3D und/oder 2D visualisieren können.

Um dem Operateur und/oder dem weiteren Benutzer einen guten Überblick zu gewähren, sind die Bilderfassungseinheiten meist zu einer Längsachse des Endoskops hin abgewinkelt, um Bilder winklig zur Längsachse des Endoskops und damit einen sogenannten Schrägblick visualisieren zu können. Dazu werden meistens Endoskope eingesetzt, die einen 30°, 45°, 60° oder 75° Schrägblick aufweisen.

Bei der Realisierung von Endoskopen mit stereoskopischer Bilderfassungseinheit, die im Einsatz einen Schrägblick aufweisen sollen, ist es besonders schwierig, insbesondere die Außendurchmesser derart zu reduzieren, dass der minimalinvasive Eingriff so gering wie möglich gehalten werden kann. Weiter soll zur besseren Orientierung des Anwenders bei der 3D- und/oder 2D-Darstellung ggf. das Bild der Bilderfassungseinheit aufrichtbar sein. Während 2D-Darstellungen auch elektronisch aufgerichtet werden können, ist dies bei 3D-Darstellungen nicht möglich. Hier kann eine Bildaufrichtung zum Beispiel durch aufwändige mechanische Systeme zur Bewegung der einzelnen Bildaufnehmer realisiert werden.

Weiter gilt es am distalen Ende des Endoskops ausreichend Licht zur Beleuchtung bereitzustellen, um das Innere des Körpers des Patienten ausreichend ausleuchten zu können, um bestmögliche Bilder aufzeichnen zu können.

Zusätzlich können durch die medizinische Bildgebung der Endoskope unterschiedliche Gewebearten, beispielsweise Organe und/oder Blutgefäße und/oder Tumorgewebe, in unterschiedlichen Tiefen unter der Haut oder in der Körperhöhle visualisiert werden. Um die Gewebearten besser differenzieren zu können, werden Fluoreszenzbildgebungsverfahren eingesetzt. Dazu wird dem Patienten ein Medikament mit einem Fluoreszenzfarbstoff, insbesondere Fluorophoren, verabreicht, das sich in einer der unterschiedlichen Gewebearten ablagert. Insbesondere bei diesen Fluoreszenzbildgebungsverfahren gilt es am distalen Ende des Endoskops ausreichend Anregungslicht zur Verfügung zu stellen, um die fluoreszierenden Substanzen ausreichend anregen zu können.

In bekannten Endoskopen mit Schrägblick werden distale Prismen eingesetzt, um den Schrägblick zu realisieren. Besonders bei Endoskopen mit Schrägblick stellt die Integration von ausreichend Beleuchtungsfasern eine hohe Herausforderung dar, da durch die distalen Prismen der Raum innerhalb des Endoskops zur Integration von Beleuchtungsfasern nur sehr eingeschränkt vorhanden ist.

Überdies ist es wichtig, das distale Ende des Endoskops während des Einsatzes spülen zu können. Zur Spülung des distalen Endes des Endoskops wird zumeist ein Fluid eingesetzt, das über entsprechende Leitungen an das distale Ende des Endoskops geführt wird. Allerdings wird für die Integration der Leitungen in bekannten Endoskopen teilweise ein zusätzlicher Spülschaft benötigt, der zusätzlich auf das Endoskop gesteckt werden muss. Durch den zusätzlichen Schaft wird der Außendurchmesser des Endoskops unnötig vergrößert. Daher muss aufgrund des zusätzlichen Schafts ein Trokar mit einem größeren Durchmesser eingesetzt werden, der den minimalinvasiven Eingriff unnötig vergrößert.

Die DE10004264C2 zeigt eine Stereokamera, die über Gelenke vom Schaft eines Endoskops abgespreizt werden kann. Durch den Vorschub eines Instruments wird die Abspreizung gesteuert und das Instrument im Blickfeld der Kamera gehalten.

WO2014104405A1 beschreibt ein Endoskop mit einer abwinkelbaren Stereokamera, die mit einem Schubmechanismus in die abgewinkelte Position gebracht werden kann.

Ein ähnlicher Mechanismus ist auch aus der EP2123225A1 bekannt.

In der US11064867B2 ist ein Endoskop offenbart, wobei eine distale Kameraeinheit durch Vorschub eines Hohlschaftes innerhalb des Instruments nach außen abgelenkt und so in eine seitlich zum Schaft versetzte Position verfahren wird.

Ausgehend vom Stand der Technik liegt der Erfindung insbesondere, aber nicht beschränkt darauf, die Aufgabe zugrunde, ein Endoskop mit Schrägblick zu schaffen, das einem Benutzer eine intuitive Bildaufrichtung ermöglicht.

Weiter liegt der Erfindung ausgehend vom Stand der Technik insbesondere, aber nicht beschränkt darauf, die Aufgabe zugrunde, mit einem möglichst kleinen minimalinvasiven Eingriff und viel Raum beispielsweise für die Integration von Beleuchtungsfasern und/oder Fluidleitungen zur Verfügung zu stellen.

Wenigstens eine dieser Aufgaben wird erfindungsgemäß durch eine Endoskopvorrichtung und ein System mit einer Endoskopvorrichtung gelöst, wie sie hierin beschrieben und in den Ansprüchen definiert sind.

Die vorliegende Erfindung sieht dazu eine Endoskopvorrichtung mit einem Schaft mit einer Schaftlängsachse vor. Die Endoskopvorrichtung umfasst weiter einen Betätigungsschaft, der parallel zur Schaftlängsachse bewegbar ist. Weiter umfasst die Endoskopvorrichtung ein Kameramodul, das schwenkbar an dem Schaft gelagert ist. Das Kameramodul umfasst weiter eine stereoskopische Bilderfassungseinheit. Das Kameramodul ist durch ein Verschieben des Betätigungsschafts relativ zum Kameramodul, insbesondere in eine Position neben dem Kameramodul, parallel zu der Schaftlängsachse, von einer Einführlage in eine Bildaufnahmelage auslenkbar. Alternativ und/oder zusätzlich ist das Kameramodul durch ein Verschieben des Kameramoduls relativ zum Betätigungsschaft, insbesondere in eine Position neben dem Betätigungsschaft, von einer Einführlage in eine Bildaufnahmelage auslenkbar. In der Bildaufnahmelage definiert das Kameramodul eine Blickrichtung, die zu der Schaftlängsachse abgewinkelt ist. Zusätzlich ist in der Bildaufnahmelage die stereoskopische Bilderfassungseinheit zu einer Bildaufrichtung mechanisch drehbar. Insbesondere ist in der Bildaufnahmelage die stereoskopische Bilderfassungseinheit zu einer Bildaufrichtung relativ zu dem Betätigungsschaft mechanisch drehbar. Beispielsweise ist in der Bildaufnahmelage die stereoskopische Bilderfassungseinheit zu einer Bildaufrichtung relativ zu dem Betätigungsschaft drehbar gelagert. Die stereoskopische Bilderfassungseinheit ist insbesondere um eine zu ihrer Blickrichtung oder zur ihrer optischen Achse parallele, oder mit der Blickrichtung oder der optischen Achse korrespondierende Drehachse drehbar.

Insbesondere wird die Endoskopvorrichtung in der Einführlage in das Körperinnere des Patienten eingeführt. Beispielweise kann die Endoskopvorrichtung durch einen Trokar in das Körperinnere eingeführt werden. In der Bildaufnahmelage weist die Endoskopvorrichtung insbesondere einen Schrägblick auf. Der Schrägblick kann beispielsweise 30° oder 45° oder 60° oder 75° betragen. Beispielsweise kann die Endoskopvorrichtung in der Bildaufnahmelage die Endoskopvorrichtung dazu eingerichtet sein, ein Bild einer Umgebung, beispielsweise von dem Inneren des Körpers des Patienten, aufzuzeichnen.

Insbesondere erstreckt sich der Schaft von einem proximalen Ende zu einem distalen Ende der Endoskopvorrichtung. Insbesondere ist der Schaft starr und/oder flexibel ausgebildet. Insbesondere ist der Schaft rund und/oder eckig ausgebildet. Vorzugsweise ist der Schaft vollständig oder teilweise aus Metall, Kunststoff und/oder Keramik ausgebildet. Die Schaftlängsachse verläuft insbesondere von dem proximalen Ende zu dem distalen Ende des Schafts. Insbesondere verläuft die Schaftlängsachse durch einen Mittelpunkt eines Querschnitts des Schafts und/oder parallel zu einer Haupterstreckungsrichtung des Schafts.

Das Kameramodul umgibt insbesondere die stereoskopische Bilderfassungseinheit. Die Bilderfassungseinheit ist insbesondere in dem Kameramodul aufgenommen und damit durch das Kameramodul und/oder durch ein Gehäuse des Kameramoduls geschützt.

Insbesondere ist das stereoskopische Kameramodul in der Einführlage am distalen Ende des Betätigungsschafts angeordnet. Das stereoskopische Kameramodul ist insbesondere beweglich an dem Schaft befestigt bzw. schwenkbar an dem Schaft gelagert. Der Betätigungsschaft ist insbesondere auf den Schaft aufgesteckt. Das stereoskopische Kameramodul umfasst eine stereoskopische Bilderfassungseinheit.

Die stereoskopische Bilderfassungseinheit kann eine erste Bilderfassungseinrichtung und eine zweite Bilderfassungseinrichtung umfassen, die jeweils in einem Spektralbereich lichtempfindlich sind. Ferner kann die stereoskopische Bilderfassungseinheit eine dritte Bilderfassungseinrichtung und/oder eine vierte Bilderfassungseinrichtung umfassen, die beispielsweise in einem weiteren Spektralbereich lichtempfindlich sind. Insbesondere ist die stereoskopische Bilderfassungseinheit ein 3D-Kamerasystem. Die erste Bilderfassungseinrichtung und/oder die zweite Bilderfassungseinrichtung können insbesondere Sensoren und Prismen umfassen, die back-to-back montiert sind. Weiter können die erste Bilderfassungseinrichtung und/oder die zweite Bilderfassungseinrichtung und/oder die dritte Bilderfassungseinrichtung und/oder die vierte Bilderfassungseinrichtung Filter und/oder eine Optik umfassen. Die Drehachse verläuft insbesondere mittig oder im Wesentlichen mittig durch die beiden Bilderfassungseinrichtungen oder mittig durch einen Teil der beiden Bilderfassungseinrichtungen, beispielsweise eine jeweils vorhandene Optik. Die Drehbarkeit der stereoskopischen Bilderfassungseinheit und/oder des ganzen Kameramoduls um die Drehachse erlaubt eine Bildaufrichtung des Stereobilds.

Mittels der ersten Bilderfassungseinrichtung und der zweiten Bilderfassungseinrichtung kann beispielsweise eine Stereobilderfassung in einem ersten Spektralbereich durchgeführt werden. Mittels der dritten Bilderfassungseinrichtung und/oder der vierten Bilderfassungseinrichtung kann beispielsweise eine Stereobilderfassung in einem zweiten Spektralbereich durchgeführt werden. Dadurch kann eine Fluoreszenzstereobilderfassung mittels der dritten Bilderfassungseinrichtung und/oder der vierten Bilderfassungseinrichtung durchgeführt werden. Um eine Fluoreszenzbildgebung zu ermöglichen, können die Bilderfassungseinrichtungen weitere Bildsensoren umfassen, die in unterschiedlichen Spektralbereichen lichtempfindlich sind. Die erste Bilderfassungseinrichtung und die zweite Bilderfassungseinrichtung können jeweils einen ersten Bildsensor umfassen, der zumindest überwiegend in dem ersten Spektralbereich, der insbesondere dem sichtbaren Licht zugeordnet ist, lichtempfindlich ist. D.h. mittels des ersten Bildsensors ist eine Bilderfassung im Wellenlängenbereich des sichtbaren Lichts durchführbar. Dies entspricht beispielsweise einer Weißlichtbilderfassung. Die dritte Bilderfassungseinrichtung und die vierte Bilderfassungseinrichtung können jeweils einen zweiten Bildsensor umfassen, der zumindest überwiegend in dem zweiten Spektralbereich, der insbesondere dem nahinfraroten Licht zugeordnet ist, lichtempfindlich ist. D.h. mittels des zweiten Bildsensors ist eine Bilderfassung im Wellenlängenbereich des nahinfraroten Lichts durchführbar.

Alternativ können die erste und zweite Bilderfassungseinrichtung sowohl im visuellen als auch im nahen Infrarot empfindlich sein und in beiden Wellenlängenbereichen Bilder, beispielsweise Fluoreszenzbilder, erfassen.

Das stereoskopische Kameramodul wird durch ein Verschieben des Betätigungsschaft, insbesondere durch ein Vorschieben des Betätigungsschafts in Richtung eines distalen Endes des Betätigungsschafts, von der Einführlage in die Bildaufnahmelage ausgelenkt. Alternativ und/oder zusätzlich kann das Kameramodul durch ein Zurückziehen des Kameramoduls in Richtung eines proximalen Endes des Kameramoduls von der Einführlage in die Bildaufnahmelage ausgelenkt werden. In anderen Worten kann das Verschieben des Betätigungsschafts parallel zu der Schaftlängsachse neben das Kameramodul, insbesondere durch ein Vorschieben des Betätigungsschafts in Richtung des Körperinneren des Patienten neben das Kameramodul und/oder durch ein Zurückschieben des Kameramoduls aus dem Körperinneren des Patienten neben den Betätigungsschaft erfolgen. Durch die Auslenkung des stereoskopischen Kameramoduls wird der Schrägblick der stereoskopischen Bilderfassungseinheit realisiert. In anderen Worten definiert durch die Auslenkung des stereoskopischen Kameramoduls das Kameramodul eine Blickrichtung, die zu der Schaftlängsachse abgewinkelt ist. Der Schrägblick kann beispielsweise 30°, 45°, 60 und/oder 75° betragen.

Insbesondere umfasst die stereoskopische Kameramodul und/oder die stereoskopische Bilderfassungseinheit eine Eingangsoptik, die eine optische Achse definiert. Die Blickrichtung verläuft insbesondere entlang der optischen Achse.

In der Bildaufnahmelage ist die stereoskopische Bilderfassungseinheit relativ zum Kameramodul und/oder relativ zum Schaft und/oder relativ zum Betätigungsschaft drehbar. Insbesondere ist die stereoskopische Bilderfassungseinheit zur Bildaufrichtung mechanisch drehbar. Beispielsweise ist dazu die stereoskopische Bilderfassungseinheit drehbar gelagert.

Die Bildaufrichtung wird zur Aufrichtung eines Bilds verwendet, das beispielsweise mit dem Kameramodul aufgezeichnet wird. Bei Drehung der Endoskopvorrichtung durch einen Anwender wird auch die Bilderfassungseinheit gedreht, was zu einer Drehung des Bildes auf einem Bildschirm führt. Eine solche Drehung kann die Orientierung für den Anwender erschweren, da die Raumrichtungen im Bild und in der Realität voneinander abweichen. So kann eine Richtung "oben", beispielsweise relativ zu einem Horizont oder zur Gravitationsrichtung, im Bild nach unten oder zur Seite gedreht sein. Um die Orientierung zu verbessern, ist es üblich normale Bilder elektronisch gegenzudrehen. Bei Stereobildern ist dies jedoch nicht möglich, da eine Drehung der endoskopischen Vorrichtung auch eine Drehung der Stereobasis bedeutet. Diese kann nicht elektronisch zurückgedreht werden. Es ist vorliegend vorteilhafterweise möglich, durch Drehung der Bilderfassungseinheit, auch ein Stereobild aufzurichten, so dass ein Stereoeindruck erhalten bleibt und die Raumrichtungen im Bild der Realität oder einer Nutzereinstellung entsprechen.

Insbesondere ist die stereoskopische Bilderfassungseinheit auch in der Einführlage des Kameramoduls drehbar gelagert ist. Die stereoskopische Bilderfassungseinheit kann unabhängig von der Bildaufnahmelage drehbar sein. Damit ist es möglich, die Bildaufrichtung der stereoskopischen Bilderfassungseinheit von der Bildaufnahmelage und/oder dem Schrägblick des Endoskops zu entkoppeln.

Um eine einfache bzw. leichtgängige Drehbarkeit der Bilderfassungseinheit zu gewährleisten, kann die Bilderfassungseinheit innerhalb des Kameramoduls drehbar gelagert sein. Beispielsweise kann aber auch die stereoskopische Bilderfassungseinheit mit dem Kameramodul zusammen drehbar gestaltet sein. Das Kameramodul kann in diesem Fall, insbesondere in der Bildaufnahmekonfiguration, an dem Betätigungsschaft gelagert sein.

Zur einfachen Bedienung kann die Bilderfassungseinheit insbesondere zur Bildaufrichtung mittels eines Kabels drehbar sein. Das Kabel kann insbesondere ein Torsionskabel und/oder ein Versorgungskabel sein. Das Torsionskabel kann beispielsweise auch direkt an dem Versorgungskabel entlanggeführt werden und/oder das Versorgungskabel verstärken und/oder das Versorgungskabel ummanteln. Das Kabel kann mit einer Elektronikeinheit, insbesondere einer Kamerakontrolleinheit (CCU), verbunden sein bzw. das Kabel kann das Kameramodul mit der Elektronikeinheit verbinden. Die Drehung des Kabels kann beispielsweise durch ein Getriebe erfolgen, das beispielsweise elektrisch angetrieben ist. Die Drehung des Kabels kann auch manuell, beispielsweise durch den Benutzer, erfolgen. Alternativ und/oder zusätzlich kann das Kabel auch zusammen mit der Elektronikeinheit gedreht werden.

Damit die Endoskopvorrichtung möglichst platzsparend in das Körperinnere eingeführt werden kann, kann das Kabel in der Einführlage in den Schaft aufgenommen sein. Dadurch wird es möglich, Trokare mit möglichst geringem Durchmesser zu verwenden, um den minimalinvasiven Eingriff so klein wie möglich zu halten. Weiter kann der Schaft beispielsweise einen Schlitz aufweisen, in den das Kabel aufgenommen werden kann. In der Bildaufnahmelage kann das Kabel insbesondere aus dem Schaft herausragen. Dadurch ist das Kabel sehr leichtgängig drehbar und damit die Bildaufrichtung sehr leichtgängig einstellbar.

In der Bildaufnahmelage kann der Betätigungsschaft das stereoskopische Kameramodul zumindest teilweise aufnehmen. Insbesondere nimmt der Betätigungsschaft das stereoskopische Kameramodul auf, um den Schrägblick der stereoskopischen Bilderfassungseinheit zu realisieren. Darüber hinaus kann insbesondere zur Realisierung des Schrägblicks in der Bildaufnahmelage eine Flächennormale einer distalen Endfläche des Betätigungsschafts winklig bzw. abgewinkelt zu der Schaftlängsachse sein. Beispielsweise verläuft die Flächennormale der distalen Endfläche des Betätigungsschafts parallel zur optischen Achse des Kameramoduls.

Um beispielsweise die Verschmutzung zwischen dem Kameramodul und dem Betätigungsschaft zu reduzieren, kann insbesondere in der Bildaufnahmelage ein distales Ende des Betätigungsschafts bündig mit einem distalen Ende des Kameramoduls abschließen. Bündig bedeutet insbesondere, dass geringfügige Abweichungen zwischen dem distalen Ende des Betätigungsschafts und dem distalen Ende des Kameramoduls gestattet sind.

Der Betätigungsschaft kann insbesondere eine Aufnahme aufweisen, in die das Kameramodul zumindest teilweise aufgenommen ist. Das Kameramodul kann insbesondere in der Bildaufnahmelage auf die Aufnahme gedrückt werden oder gegen diese vorgespannt sein. Die Aufnahme kann weiter insbesondere bezüglich einer Längsachse des Betätigungsschafts und/oder der Schaftlängsachse abgewinkelt sein. Weiter kann die Aufnahme an eine Form des Kameramoduls angepasst sein. Beispielsweise kann die Aufnahme eine halbrunde und/oder eckige und/oder U-förmige Vertiefung ausbilden und/oder aufweisen. Zusätzlich kann das Kameramodul bezüglich der Aufnahme drehbar gelagert sein.

Damit das Kameramodul sicher auf dem Betätigungsschaft und/oder der Aufnahme aufliegt, kann, insbesondere in der Bildaufnahmelage, das Kameramodul auf dem Betätigungsschaft, insbesondere auf der Aufnahme des Betätigungsschafts, aufliegen. Zudem kann, insbesondere in der Bildaufnahmelage, das Kameramodul gegen den Betätigungsschaft, insbesondere gegen die Aufnahme des Betätigungsschafts, vorgespannt sein und insbesondere gegen diesen gedrückt werden.

Um die Bewegung zwischen dem Kameramodul und dem Schaft zu definieren, kann das Kameramodul durch eine Schwenkvorrichtung an dem Schaft schwenkbar gelagert sein.

Um eine sichere Positionierung des Kameramoduls zu realisieren, kann das Kameramodul durch die Schwenkvorrichtung gegen den Betätigungsschaft, insbesondere gegen die Aufnahme des Betätigungsschafts, gedrückt werden.

Um die Bewegung zwischen dem Kameramodul und dem Schaft zu definieren und leichtgängiges Schwenken zu gewährleisten, weist die Schwenkvorrichtung ein erstes Drehgelenk und ein zweites Drehgelenk auf. Das erste Drehgelenk und das zweite Drehgelenk können voneinander beabstandet sein. Insbesondere ist das erste Drehgelenk mit dem Schaft und das zweite Drehgelenk mit dem Kameramodul verbunden.

Um eine sichere Positionierung des Kameramoduls zu realisieren, kann das erste Drehgelenk und/oder das zweite Drehgelenk eine Torsionsfeder aufweisen, durch die das Kameramodul in der Bildaufnahmelage auf den Betätigungsschaft gedrückt wird.

Um das Körperinnere ausleuchten zu können, kann der Betätigungsschaft zumindest eine lichtauskoppelnde Fläche aufweisen, die dazu eingerichtet ist, Beleuchtungslicht nach distal auszukoppeln. Die lichtauskoppelnde Fläche kann insbesondere durch Beleuchtungsfasern und/oder Optiken und/oder LEDs gebildet werden.

Der Betätigungsschaft kann zumindest einen Lichtleiter und/oder Beleuchtungsfasern, und/oder zumindest eine Fluidleitung umfassen, die sich entlang einer Längsachse des Betätigungsschafts erstrecken. Dadurch, dass sich die Fluidleitungen entlang der Längsachse des Betätigungsschafts erstecken, werden diese insbesondere nicht gebogen und weisen dadurch eine höhere Haltbarkeit auf. Weiter entstehen dadurch insbesondere weniger Reibungsverluste des Fluids und es kann eine zumindest weitestgehend laminare Strömung des Fluids realisiert werden.

Zusätzlich kann der Betätigungsschaft zumindest eine Öffnung für einen Lichtleiter aufweisen, um Beleuchtungslicht nach distal auszukoppeln. Alternativ und/oder zusätzlich kann der Betätigungsschaft zumindest eine Öffnung für eine Fluidleitung aufweisen, um insbesondere mittels eines Fluids, insbesondere Luft, insbesondere Druckluft, und/oder Gas, insbesondere Sterilgas, und/oder Wasser das distale Ende des Betätigungsschafts reinigen zu können. Insbesondere wird durch das Fluid das Kameramodul, insbesondere die Bilderfassungseinheit, und/oder die lichtauskoppelnde Fläche und/oder die zumindest eine Öffnung des Lichtleiters und/oder zumindest eine Öffnung der Fluidleitungen gereinigt. Weiter kann alternativ und/oder zusätzlich durch die wenigstens eine Fluidleitung ein Fluid, insbesondere Druckluft, und/oder Gas, insbesondere Sterilgas, und/oder Wasser und/oder Blut und/oder andere Körperflüssigkeiten, von dem distalen Ende abgesaugt werden. Insbesondere können der Lichtleiter und/oder die Fluidleitung durch zumindest eine Öffnung aus dem Betätigungsschaft austreten. Insbesondere kann aus der Öffnung das Beleuchtungslicht aus dem Lichtleiter und/oder das Fluid aus der Fluidleitung aus dem Betätigungsschaft austreten und/oder es kann durch die Öffnung das Fluid von dem distalen Ende abgesaugt werden.

Zur kostengünstigen Herstellung ist insbesondere der Betätigungsschaft aus Kunststoff gebildet. Darüber hinaus kann die wenigstens eine Fluidleitung des Betätigungsschafts mit wenigstens einer Zuleitung verbunden sein. Durch die Verbindung der wenigstens einen Zuleitung mit der wenigstens einen Fluidleitung des Betätigungsschafts können die Zuleitungen an einem proximalen Ende der wenigstens einen Zuleitung flexible ausgestaltet sein. Dadurch kann die wenigstens eine Zuleitung direkt bzw. ohne gesonderte Schnittstelle mit einer Pumpeneinheit und/oder Insufflationseinheit verbunden werden.

Weiter kann zumindest eine Komponente des Betätigungsschafts und/oder der Betätigungsschaft als Einmalteil ausgebildet sein, um Reinigungskosten reduzieren zu können.

Um zu gewährleisten, dass nahezu kein Fluid an einer ungewünschten Stelle aus der Endoskopvorrichtung austritt, kann der Betätigungsschaft durch ein Dichtelement, insbesondere mittels O-Ring zum Schaft abdichten.

Um den Betätigungsschaft besser an die Funktionen, die der Betätigungsschaft erfüllt, anpassen zu können bzw. um einzelne Teile des Betätigungsschaft verbessert an ihre Funktionen und/oder Verwendung anpassen zu können, kann insbesondere der Betätigungsschaft einen Aufnahmeschaft aufweisen. Insbesondere weist der Aufnahmeschaft einen Kanal auf, in den der Schaft einschiebbar ist.

Um den Betätigungsschaft zusätzlich besser an die jeweiligen Funktionen, die der Betätigungsschaft erfüllt, anpassen zu können bzw. um einzelne Teile des Betätigungsschaft verbessert an ihre Funktionen und/oder Verwendung anpassen zu können, kann der Betätigungsschaft insbesondere zusätzlich oder alternativ zu dem Aufnahmeschaft einen Beleuchtungsstab umfassen. Insbesondere kann der Beleuchtungsstab zumindest eine lichtauskoppelnde Fläche, insbesondere lichtauskoppelnde Beleuchtungsfasern und/oder eine lichtauskoppelnde Optik, aufweisen, die dazu eingerichtet ist, Beleuchtungslicht nach distal auszukoppeln.

Zur Realisierung einer einfachen modularen Bauweise des Betätigungsschafts weist der Aufnahmeschaft einen Kanal auf, in den der Beleuchtungsstab einschiebbar ist.

Zur Bereitstellung von Beleuchtungslicht kann der Beleuchtungsstab zumindest einen Lichtleiter umfassen, der sich entlang einer Längsachse des Beleuchtungsstabs erstreckt. Beispielsweise kann der Lichtleiter mit dem lichtauskoppelnden optischen Element gekoppelt sein.

Zur kostengünstigen Herstellung kann der Aufnahmeschaft aus Kunststoff ausgebildet sein. Insbesondere kann der Aufnahmeschaft als Einmalteil ausgebildet sein, damit dieser nicht aufwändig gereinigt werden muss.

Zur Verlängerung der Lebensdauer kann der Beleuchtungsstab zumindest an einem distalen Ende oder teilweise aus Metall ausgebildet sein. Insbesondere ist der Beleuchtungsstab derart ausgestaltet, dass der Beleuchtungsstab mehrfach verwendbar und insbesondere autoklavierbar ist.

Um den Lichtleiter flexibel an eine Lichtquelle anschließen zu können, kann der Lichtleiter außerhalb des Körperinneren des Patienten bzw. außerhalb des Trokars an einem proximalen Ende des Beleuchtungsstabs und/oder des Betätigungsschafts in einem flexiblen Schlauch zu der Lichtquelle weitergeführt werden. Insbesondere kann die Lichtleitfaser durchgehend von dem distalen Ende zu dem proximalen Ende des Beleuchtungsstabs und/oder des Betätigungsschafts verlaufen. Beispielsweise verläuft die Lichtleitfaser von dem distalen Ende zu dem proximalen Ende des Beleuchtungsstab und/oder des Betätigungsschafts ohne Unterbrechung und/oder ohne eine zusätzliche Schnittstelle. Durch die Vermeidung der Schnittstelle bzw. dadurch, dass die Lichtleitfaser ohne Unterbrechung und/oder durchgehend von dem proximalen Ende zu dem distalen Ende des Beleuchtungsstabs und/oder des Betätigungsschafts verläuft, kann der Lichtverlust stark reduziert werden. In diesem Zusammenhang haben die Erfinder erkannt, dass sich der Lichtverlust im Vergleich zu ähnlichen Lichtleitfasern, die durch eine Schnittstelle miteinander verbunden werden, um 30 % reduzieren kann.

Um die Lebensdauer und die Lichtausbeute des Lichtleiters zu erhöhen, kann zudem der Lichtleiter an einem distalen Ende des Beleuchtungsstabs und/oder des Betätigungsschafts in die Blickrichtung vorgebogen und/oder poliert sein. D.h., es kann insbesondere vorgesehen sein, für jede Blickrichtung einen unterschiedlichen Beleuchtungsstab und/oder Betätigungsschaft vorzusehen, der flexibel austauschbar ist. Insbesondere können die verschiedenen austauschbaren Beleuchtungsstäbe und/oder Betätigungsschäfte flexibel mit einem gleichbleibenden Schaft, an dem das Kameramodul schwenkbar gelagert ist, verwendet werden. Weiter kann der Lichtleiter, insbesondere an dem distalen Ende des Beleuchtungsstabs und/oder des Betätigungsschafts, in den Beleuchtungsstab und/oder den Betätigungsschaft verklebt sein.

Um zu verhindern, dass das Fluid zwischen dem Schaft und dem Aufnahmeschaft austritt, kann der Aufnahmeschaft zumindest ein Dichtelement, insbesondere einen O-Ring, aufweisen, der zum Schaft abdichtet. Um zu verhindern, dass das Fluid zwischen dem Beleuchtungsstab und dem Aufnahmeschaft austritt, kann der Aufnahmeschaft zumindest ein Dichtelement, insbesondere einen O-Ring, aufweisen, das zum Beleuchtungsstab abdichtet.

Um eine einfache modulare Bauweise zu realisieren, kann insbesondere der Aufnahmeschaft die Aufnahme aufweisen, in die das Kameramodul zumindest teilweise aufgenommen ist. Um das Kameramodul, insbesondere in der Bildaufnahme, zu definieren, kann das Kameramodul insbesondere auf die Aufnahme gedrückt werden.

Um den Schrägblick des Kameramoduls und/oder des Bilderfassungseinheit zu realisieren, kann die Aufnahme zu einer Längsachse des Betätigungsschafts und/oder zu einer Längsachse des Aufnahmeschafts abgewinkelt sein. Beispielsweise kann die Aufnahme als eine halbrunde und/oder eckige und/oder U-förmige Vertiefung ausgebildet sein, um das Kameramodul sicher aufnehmen zu können. Alternativ oder zusätzlich kann dazu die Aufnahme an eine Form der Bilderfassungseinheit angepasst sein.

Um eine einfache und/oder benutzerfreundliche Bildaufrichtung zu gewährleisten, kann insbesondere das Kameramodul bezüglich der Aufnahme drehbar gelagert sein.

Um die Position der Kamera in der Bildaufnahme zu definieren, kann insbesondere in der Bildaufnahmelage das Kameramodul auf dem Aufnahmeschaft aufliegen. Insbesondere um den Schrägblick der Endoskopvorrichtung zu realisieren, kann eine Flächennormale einer distalen Endfläche des Aufnahmeschafts und/oder des Beleuchtungsstabs winklig zu der Schaftlängsachse verlaufen. Insbesondere kann dazu weiter oder alternativ in der Bildaufnahmelage ein distales Ende des Aufnahmeschafts und/oder des Beleuchtungsstabs bündig mit einem distalen Ende des Kameramoduls abschließen.

Insbesondere kann der Aufnahmeschaft zumindest eine Fluidleitung umfassen, die sich entlang einer Längsachse des Aufnahmeschafts erstreckt. Dadurch wird verhindert, dass die Fluidleitung in dem Aufnahmeschaft gebogen wird. Dies führt zu einer Erhöhung der Lebensdauer der Fluidleitung und reduziert die Reibungsverluste des Fluids, das durch die Fluidleitung geleitet wird. Der Aufnahmeschaft kann insbesondere zumindest eine Öffnung für eine Fluidleitung aufweisen. Beispielsweise kann die Fluidleitung und/oder das Fluid durch zumindest eine Öffnung aus dem Aufnahmeschaft austreten.

Zusätzlich umfasst die Erfindung ein System mit einer Endoskopvorrichtung, wie sie hierin beschrieben und in den Ansprüchen definiert ist, und einen weiteren Betätigungsschaft, der anstelle des Betätigungsschafts verwendbar ist. Um mehrere Blickrichtungen und/oder Schrägblicke zu realisieren, kann der Betätigungsschaft insbesondere eine erste Bildaufnahmelage mit einer ersten Blickrichtung definieren. Weiter kann insbesondere der weitere Betätigungsschaft eine zweite Bildaufnahmelage mit einer von der ersten Blickrichtung verschiedenen zweiten Blickrichtung definieren. Es können also in dem System zur Realisierung von unterschiedlichen Blickrichtungen und/oder Schrägblicken mehrere Betätigungsschäfte eingesetzt werden, die jeweils einen unterschiedlichen Schrägblick und/oder eine unterschiedliche Blickrichtung liefern.

Zudem umfasst die Erfindung ein System mit einer Endoskopvorrichtung, wie sie hierin beschrieben und in den Ansprüchen definiert ist, die einen Betätigungsschaft mit einem Aufnahmeschaft und einem Beleuchtungsstab umfasst. Der Betätigungsstab umfasst insbesondere wenigstens einen weiteren Aufnahmeschaft, der anstelle des Aufnahmeschafts verwendbar ist. Um mehrere Blickrichtungen und/oder Schrägblicke zu realisieren, kann der Aufnahmeschaft insbesondere eine erste Bildaufnahmelage mit einer ersten Blickrichtung definieren. Weiter kann insbesondere der weitere Aufnahmeschaft eine zweite Bildaufnahmelage mit einer von der ersten Blickrichtung verschiedenen zweiten Blickrichtung definieren. Es können also in dem System zur Realisierung von unterschiedlichen Blickrichtungen und/oder Schrägblicke mehrere unterschiedliche Aufnahmeschäfte eingesetzt werden, die jeweils einen unterschiedlichen Schrägblick und/oder eine unterschiedliche Blickrichtung liefern. Der Beleuchtungsstab hingegen kann insbesondere derart ausgestaltet sein, sodass dieser mit den unterschiedlichen Aufnahmeschäften eingesetzt werden kann. Alternativ kann aber auch für jeden Aufnahmeschaft ein weiterer Beleuchtungsstab verwendet werden, der insbesondere eine lichtauskoppelnde Fläche aufweist, die in die Blickrichtung abstrahlt, die mit dem jeweiligen Aufnahmeschaft realisiert wird.

Im Folgenden wird die vorliegende Erfindung anhand der beigefügten Figuren beispielhaft beschrieben. Die Zeichnung, die Beschreibung und die Ansprüche enthalten zahlreiche Merkmale in Kombination. Der Fachmann wird die Merkmale zweckmäßigerweise auch einzeln betrachten und im Rahmen der Ansprüche sinnvoll in Kombination verwenden.

Falls von einem bestimmten Objekt mehr als ein Exemplar vorhanden ist, ist ggf. nur eines davon in den Figuren und in der Beschreibung mit einem Bezugszeichen versehen. Die Beschreibung dieses Exemplars kann entsprechend auf die anderen Exemplare von dem Objekt übertragen werden. Sind Objekte insbesondere mittels Zahlenwörtern, wie beispielsweise erstes, zweites, drittes Objekt etc. benannt, dienen diese der Benennung und/oder Zuordnung von Objekten. Demnach können beispielsweise ein erstes Objekt und ein drittes Objekt, jedoch kein zweites Objekt umfasst sein. Allerdings könnten anhand von Zahlenwörtern zusätzlich auch eine Anzahl und/oder eine Reihenfolge von Objekten ableitbar sein.

Es zeigen:
- Figur 1: eine beispielhafte Ausführungsform einer erfindungsgemäßen Endoskopvorrichtung;
- Figur 2: eine beispielhafte Ausführungsform einer erfindungsgemäßen Endoskopvorrichtung in einer Bildaufnahmelage;
- Figur 3: eine distale Ansicht einer beispielhaften Ausführungsform einer erfindungsgemäßen Endoskopvorrichtung in der Bildaufnahmelage;
- Figur 4: eine beispielhafte Ausführungsform eines erfindungsgemäßen stereoskopischen Kameramoduls, das schwenkbar an dem Schaft gelagert ist;
- Figur 5: eine distale Ansicht der beispielhaften Ausführungsform, die in Figur 4 dargestellt ist;
- Figur 6: eine beispielhafte Ausführungsform eines erfindungsgemäßen Beleuchtungsstabs;
- Figur 7: eine distale Ansicht der beispielhaften Ausführungsform, die in Figur 6 dargestellt ist;
- Figur 8: eine beispielhafte Ausführungsform eines erfindungsgemäßen Aufnahmeschafts;
- Figur 9: eine distale Ansicht einer beispielhaften Ausführungsform eines erfindungsgemäßen Aufnahmeschafts.

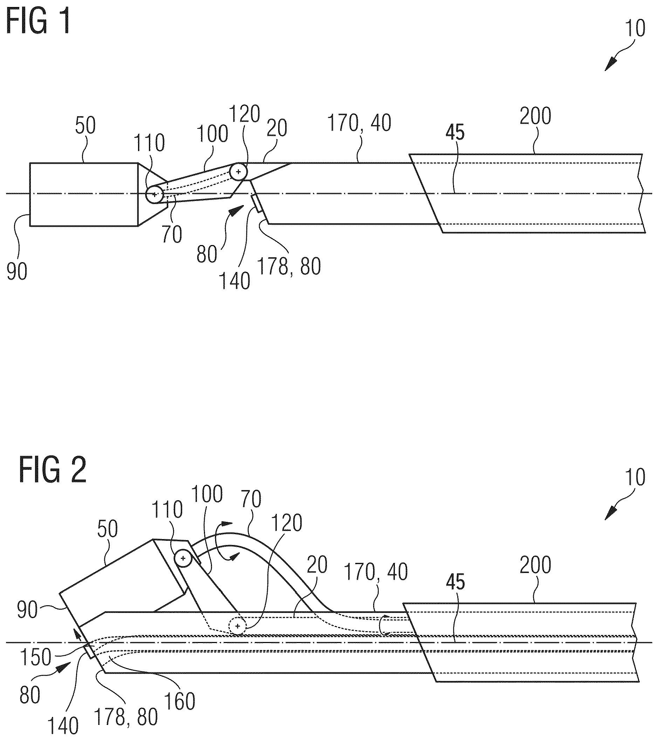

Figur 1 zeigt eine beispielhafte Ausführungsform einer erfindungsgemäßen Endoskopvorrichtung 10 in einer Einführlage, in der die Endoskopvorrichtung 10, beispielsweise durch einen Trokar 200, in ein Körperinneres eines Patienten eingeführt werden kann. Figur 2 zeigt eine beispielhafte Ausführungsform einer erfindungsgemäßen Endoskopvorrichtung 10 in einer Bildaufnahmelage des Kameramoduls 50.

Die Endoskopvorrichtung 10 umfasst in beiden Ausführungsformen, die in Figur 1 und Figur 2 dargestellt sind, ein stereoskopisches Kameramodul 50, das schwenkbar an einem Schaft 20 angeordnet ist, und einen Betätigungsschaft 40. Der Schaft 20 weist eine Schaftlängsachse 30 auf, die sich von einem proximalen Ende zu einem distalen Ende des Schafts 20 erstreckt. Beispielsweise kann der Schaft 20 durch den Betätigungsschaft 40 hindurchgeführt sein. Beispielsweise kann das Kameramodul 50 durch eine Schwenkvorrichtung 100 schwenkbar an dem Schaft 20 angeordnet sein. Insbesondere umfasst die Schwenkvorrichtung 100 ein erstes Drehgelenk 110 und ein zweites Drehgelenk 120 sowie einen Steg, der das erste Drehgelenk 110 und das zweite Drehgelenk 120 miteinander verbindet. Das erste Drehgelenk 110 kann beispielsweise mit dem Kameramodul 50 verbunden sein und das zweite Drehgelenk 120 kann beispielsweise mit dem Schaft 20 verbunden sein.

Das zweite Drehgelenk 120 kann beispielsweise an einer Seite des Schafts 20 und/oder an beiden Seiten des Schafts 20 gelagert sein. Das zweite Drehgelenk 120 kann beispielsweise eine Welle umfassen, die durch den Schaft 20 hindurchgeführt ist und mit dem Steg fest verbunden ist. Das zweite Drehgelenk 120 kann also durch die Welle zu dem Schaft 20 drehbar gelagert sein. Insbesondere erfolgt die Lagerung mittels eines Gleitlagers zwischen dem Schaft 20 und der Welle.

Das erste Drehgelenk 110 kann beispielsweise an einer Seite das Kameramoduls 50 und/oder an beiden Seiten des Kameramoduls 50 gelagert sein. Das erste Drehgelenk 110 kann beispielsweise eine Welle umfassen, die durch das Kameramodul 50 hindurchgeführt ist und mit dem Steg fest verbunden ist. Das erste Drehgelenk 110 kann also durch die Welle zu dem Kameramodul 50 drehbar gelagert sein. Insbesondere erfolgt die Lagerung mittels eines Gleitlagers zwischen dem Kameramodul 50 und der Welle.

Zusätzlich kann das Kameramodul 50 mit einem Kabel 70 verbunden sein. Über das Kabel 70 kann das Kameramodul, insbesondere zur Bildaufrichtung, in der Bildaufnahmelage gedreht werden. Insbesondere kann das Kabel 70 ein Versorgungskabel und ein Torsionskabel umfassen. Beispielsweise kann das Torsionskabel das Versorgungskabel ummanteln.

Damit die Schwenkvorrichtung 100 das Kameramodul 50 auf den Betätigungsschaft 40 drücken kann, kann das erste Drehgelenk 110 und/oder das zweite Drehgelenk 120 eine Torsionsfeder aufweisen. Die Torsionsfeder kann durch die Auslenkung der Schwenkvorrichtung 100 durch das Verschieben des Betätigungsschafts 40 neben das Kameramodul 50 vorgespannt werden. Insbesondere wird die Torsionsfeder derart vorgespannt, sodass die Torsionsfeder ein Rotationsmoment auf die Schwenkvorrichtung 100 ausübt. Durch das Rotationsmoment kann das Kameramodul 50 auf den Betätigungsschaft 40 gedrückt werden.

Der Betätigungsschaft 40 und/oder der später beschriebene Aufnahmeschaft 170 können beispielsweise eine Aufnahme 175 aufweisen, in die das Kameramodul 50 aufgenommen werden kann, um die Bildaufnahmelage des Kameramoduls 50 zu definieren. Insbesondere kann die Aufnahme 175 eine Runde und/oder eckige Geometrie aufweisen. Vorzugsweise ist die Aufnahme 175 an die Form des Kameramoduls 50 angepasst.

Durch den Betätigungsschaft 40 kann ein Lichtleiter 150 hindurchgeführt sein, dessen Licht über eine lichtauskoppelnde Fläche 140 aus dem Betätigungsschaft 40 austritt und der sich entlang einer Längsachse 45 des Betätigungsschafts 40 erstreckt. Die lichtauskoppelnde Fläche 140 ist beispielsweise an dem distalen Ende des Betätigungsschafts 40 angeordnet. Die lichtauskoppelnde Fläche 140 kann alternativ und/oder zusätzlich durch beispielsweise eine LED gebildet werden. Vorzugsweise ist die lichtauskoppelnde Fläche 140 in einem ähnlichen und/oder identischen Winkel zu der Schaftlängsachse 30 abgewinkelt, in der das Kameramodul 50 in der Bildaufnahmelage zu der Schaftlängsachse 30 abgewinkelt ist. D. h., die lichtauskoppelnde Fläche 140 ist vorzugsweise in einer Richtung ausgerichtet, die dem Schrägblick der Endoskopvorrichtung 10 in der Bildaufnahmelage entspricht.

Weiter kann wenigstens eine Fluidleitung 160 durch den Betätigungsschaft 40 hindurchgeführt sein und sich entlang der Längsachse 45 des Betätigungsschafts 40 erstrecken. Durch die wenigstens eine Fluidleitung 160 kann ein Fluid, beispielsweise Wasser und/oder Luft und/oder Gas von einem proximalen Ende des Betätigungsschafts 40 an ein distales Ende des Betätigungsschafts 40 geleitet werden.

Insbesondere tritt das Fluid aus wenigstens einer Öffnung aus dem Betätigungsschaft 40 aus. Die wenigstens eine Öffnung ist insbesondere an dem distalen Ende des Betätigungsschafts 40 angeordnet. Beispielsweise und/oder vorzugsweise ist das distale Ende des Betätigungsschafts 40 in einem ähnlichen und/oder identischen Winkel zu der Schaftlängsachse 30 abgewinkelt, in der das Kameramodul 50 in der Bildaufnahmelage zu der Schaftlängsachse 30 abgewinkelt ist. D. h., die wenigstens eine Öffnung ist vorzugsweise in einer Richtung ausgerichtet, die dem Schrägblick der Endoskopvorrichtung 10 in der Bildaufnahmelage entspricht.

Der Betätigungsschaft 40 kann einen Aufnahmeschaft 170 und einen Beleuchtungsstab 180 umfassen. Der Betätigungsschaft 40 kann außerdem als Aufnahmeschaft 170 ausgebildet sein. Der Aufnahmeschaft 170 und der Beleuchtungsstab 180 können voneinander getrennte Bauteile sein. Insbesondere kann der Beleuchtungsstab 180 durch den Aufnahmeschaft 170 hindurchgeführt sein bzw. es kann der Beleuchtungsstab 180 durch den Aufnahmeschaft 170 hindurch gesteckt werden. Der Aufnahmeschaft 170 und der Beleuchtungsstab 180 können aber auch ein Bauteil bzw. ein zusammenhängendes Bauteil bilden. Weiter kann der Aufnahmeschaft 170 eine Kavität zur Aufnahme des Schafts 20 aufweisen.

Der Aufnahmeschaft 170 kann dazu eingerichtet sein, das Kameramodul 50 in der Bildaufnahmelage aufzunehmen und damit den Schrägblick des Kameramoduls 50 zu realisieren. Darüber hinaus kann der Aufnahmeschaft 170 die wenigstens eine Fluidleitung 160 umfassen sowie die wenigstens eine Öffnung, für die wenigstens eine Fluidleitung aufweisen. Beispielsweise ist der Aufnahmeschaft 170 aus Kunststoff geformt. Zudem kann der Aufnahmeschaft 170 derart produziert sein, sodass dieser nur zur einmaligen Verwendung geeignet ist.

Der Beleuchtungsstab 180 kann den wenigstens einen Lichtleiter 150 umfassen. Der Lichtleiter 150 kann von einem proximalen Ende des Beleuchtungsstabs 180 zu einem distalen Ende des Beleuchtungsstabs 180 verlaufen und in Form der lichtauskoppelnden Fläche 140 aus dem distalen Ende 178 des Aufnahmeschaft 180 austreten. Beispielsweise ist der Beleuchtungsstab 180 zumindest teilweise aus einem beständigen Material, beispielsweise Metall, geformt und/oder mit dem beständigen Material ummantelt.

Wie in Figur 1 dargestellt, kann zur Einführung der Endoskopvorrichtung 10 durch den Trokar das Kameramodul 50 an einem distalen Ende des Betätigungsschafts 40 angeordnet sein. Dadurch kann die Endoskopvorrichtung 10 durch den Trokar 200 in das Körperinnere des Patienten eingeführt werden, obwohl das Kameramodul 50 und der Betätigungsschaft 40 beide einen Innendurchmesser des Trokars ausfüllen.

Wie in Figur 2 dargestellt, kann durch ein Verschieben und/oder durch ein Vorschieben des Betätigungsschafts 40 in Richtung des Körperinneren des Patienten neben das Kameramodul 50 und/oder durch ein Zurückschieben des Kameramoduls 50 aus dem Körperinneren des Patienten neben den Betätigungsschaft 40, das Kameramodul 50 durch die Schwenkvorrichtung 100 derart ausgelenkt werden, sodass das Kameramodul 50 in der Bildaufnahmelage auf den Betätigungsschaft 40 gedrückt wird. Insbesondere schließt ein distales Ende 90 des Kameramoduls 50 in der Bildaufnahmelage mit einem distalen Ende 80 des Betätigungsschafts 40 bündig ab. Beispielsweise kann das distale Ende 90 des Kameramoduls 50 in der Bildaufnahmelage insbesondere parallel zu dem distalen Ende 80 des Betätigungsschafts 40 verlaufen. Das Kameramodul 50 ist relativ zum Betätigungsschaft 40 drehbar gelagert, wie dies durch einen Pfeil angedeutet ist. Das Kameramodul 50 kann manuell oder durch einen Motor gedreht werden, beispielsweise Drehung des mit dem Kameramodul 50 verbundenen Kabels 70, um die Stereobasis des Kameramoduls zu drehen und ein Bild aufzurichten.

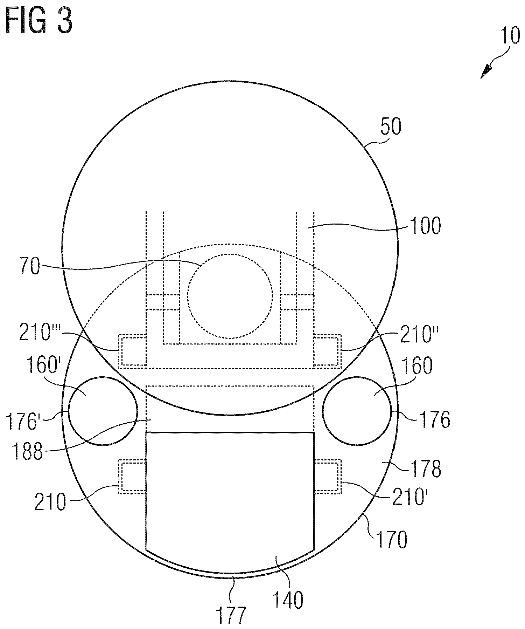

Figur 3 zeigt eine distale Ansicht einer beispielhaften Ausführungsform einer erfindungsgemäßen Endoskopvorrichtung 10 in der Bildaufnahmelage. Die Figur 3 zeigt das distale Ende 90 des Kameramoduls 50 und das distale Ende 80 des Betätigungsschafts 40. In der beispielhaften Ausführungsform, die in Figur 3 dargestellt ist, umfasst der Betätigungsschaft 40 den Aufnahmeschaft 170 und den Beleuchtungsstab 180. Daher zeigt die Figur 3 auch das distale Ende 178 des Aufnahmeschafts 170 und das distale Ende 188 des Beleuchtungsstabs 180.

Das Kameramodul 50 ist an der proximalen Seite durch die Schwenkvorrichtung 100 mit dem Schaft verbunden. Ferner ist das Kameramodul an der proximalen Seite mit dem Kabel 70 verbunden. In der distalen Ansicht der beispielhaften Ausführungsform der Endoskopvorrichtung 10, die in Figur 3 dargestellt ist, ist die Schwenkvorrichtung 100 und das Kabel 70 hinter dem Kameramodul 50 angeordnet und daher durch das Kameramodul 50 verdeckt. Daher ist die Schwenkvorrichtung 100 und das Kabel 70 gestrichelt dargestellt.

Der Beleuchtungsstab 180 kann insbesondere in den Aufnahmeschaft 170 eingesteckt sein. Damit der Beleuchtungsstab 180 möglichst ganz mit dem Lichtleiter und/oder wenigstens einem Lichtleiter ausgefüllt werden kann, kann der Beleuchtungsstab 180 an dessen Rand Führungen, insbesondere Führungsnasen, 210, 210', 210", 210‴ aufweisen, mittels derer der Beleuchtungsstab 180 in dem Aufnahmeschaft 170 fixiert werden kann. An dem distalen Ende 188 des Beleuchtungsstabs 180 ist eine lichtauskoppelnde Fläche 140 angeordnet.

Das distale Ende 178 des Aufnahmeschafts 170 weist Öffnungen 176 und 176' für die Fluidleitungen 160 und 160' auf. Insbesondere sind die Öffnungen 176 und 176' zwischen den Führungen 210, 210', 210", 210‴ angebracht. Vorzugsweise sind die Öffnungen 176 und 176' derart angeordnet, sodass diese das Kameramodul 50, insbesondere eine Bilderfassungseinheit (nicht dargestellt) des Kameramoduls 50, und/oder die lichtauskoppelnde Fläche 140 reinigen kann.

Weiter weist der Aufnahmeschafft 170 eine Aufnahme 175 auf, durch die das Kameramodul 150 in der Bildaufnahmelage aufgenommen wird. Vorzugsweise ist eine Form der Aufnahme 175 an eine Form des Kameramoduls 150 angepasst. Beispielsweise kann die Aufnahme 175, eine halbrunde Form und das Kameramodul 150 eine runde Form aufweisen.

Die Aufnahme 175 kann relativ zur Längsachse 45 des Betätigungsschafts 40 und/oder zur Längsachse des Aufnahmeschafts 170 abgewinkelt ausgestaltet sein.

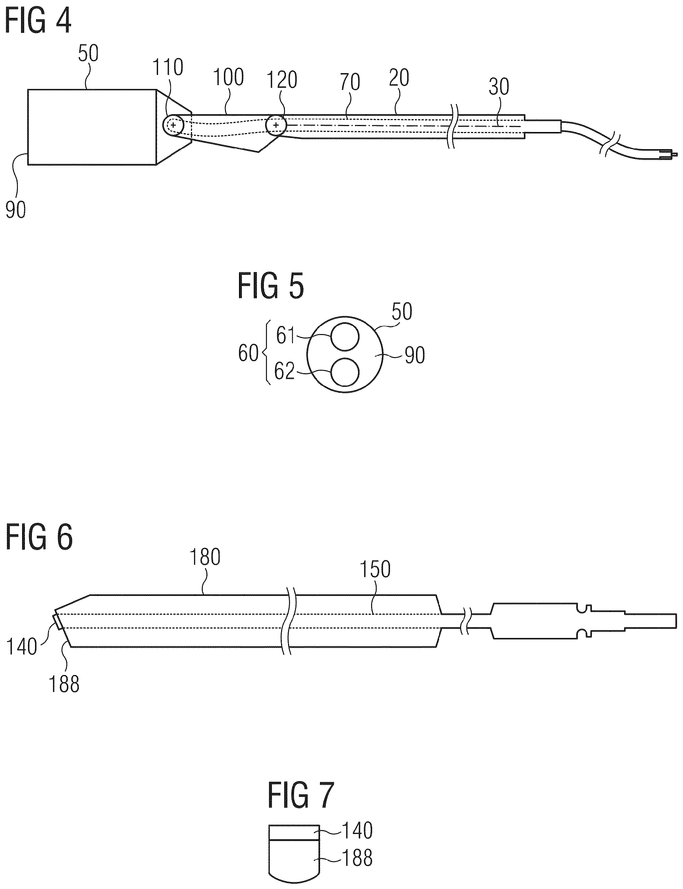

Figur 4 zeigt eine beispielhafte Ausführungsform des erfindungsgemäßen stereoskopischen Kameramoduls 50, das mittels der Schwenkvorrichtung 100 schwenkbar an dem Schaft 20 gelagert ist. Die Schwenkvorrichtung 100 kann ein erstes Drehgelenk 110 und ein zweites Drehgelenk 120 umfassen. Insbesondere ist das erste Drehgelenk 110 mit dem Kameramodul 50 und das zweite Drehgelenk 120 mit dem Schaft 20 verbunden.

Der Schaft 20 weist die Schaftlängsachse 30 auf. Das Kameramodul 50 ist mit dem Kabel 70 verbunden. Das Kabel 70 kann, wie in Figur 4 dargestellt, durch den Schaft 20 geführt sein. Dazu kann der Schaft 20 einen Schlitz oder eine beispielsweise U-förmige Aufnahme aufweisen, in denen das Kabel 70 eingeführt ist. D. h., in der Einführlage kann das Kabel 70 in den Schaft 20 aufgenommen sein und in der Bildaufnahmelage kann das Kabel 70 aus dem Schaft 20 herausragen bzw. kann das Kabel 70 durch die Auslenkung des Kameramoduls 50 in die Bildaufnahmelage aus dem Schaft 20 ausgetrieben werden. Dadurch ist das Kameramodul 50 durch das Kabel 70 in der Bildaufnahmelage leichtgängig relativ zum Betätigungsschaft 40 und zur Aufnahme 175 drehbar. Es ist aber auch möglich, dass das Kabel 70 in der Einführlage als auch in der Bildaufnahmelage durch den Schaft 20 geführt ist. Beispielsweise kann das Kabel 70 auch durch die Schwenkvorrichtung 100 geführt sein. Dadurch kann das Verletzungsrisiko an unerwünschten Stellen des Inneren des Körpers des Patienten reduziert werden.

Figur 5 zeigt eine distale Ansicht der beispielhaften Ausführungsform, die in Figur 4 dargestellt ist bzw. das distale Ende 90 des Kameramodul 50. In das Kameramodul 50 ist eine stereoskopische Bilderfassungseinheit 60 integriert. Die Bilderfassungseinheit 60 umfasst eine erste Bilderfassungseinrichtung 61 und eine zweite Bilderfassungseinrichtung 62. Die erste Bilderfassungseinrichtung 61 und die zweite Bilderfassungseinrichtung 62 können einem Stereobasisabstand voneinander beanstandet sein.

Figur 6 zeigt eine beispielhafte Ausführungsform des erfindungsgemäßen Beleuchtungsstab 180. An dem distalen Ende 188 weist der Beleuchtungsstab 180 eine lichtauskoppelnde Fläche 140 auf. Beispielsweise kann die lichtauskoppelnde Fläche 140, wie dargestellt, an dem distalen Ende 188 aus dem Beleuchtungsstab 180 herausragen. Es ist aber auch möglich, dass die lichtauskoppelnde Fläche 140 derart in den Beleuchtungsstab 180 integriert ist, sodass die lichtauskoppelnde Fläche 140 nicht aus dem Beleuchtungsstab 180 herausragt. Die lichtauskoppelnde Fläche 140 ist mit einem Lichtleiter 150 verbunden, der durch den Beleuchtungsstab 180 hindurchgeführt ist. Insbesondere weist der Lichtleiter 150 keine Unterbrechung und/oder Schnittstelle auf, damit ein Lichtmenge, die durch den Lichtleiter 150 zu der lichtauskoppelnde Fläche 140 transportiert wird, vergrößert werden kann. Das distale Ende 188 des Beleuchtungsstab 180 ist insbesondere zu einer Senkrechten der Schaftlängsachse und/oder zu einer Senkrechten einer Längsachse des Beleuchtungsstab 180 abgewinkelt. Insbesondere ist das distale Ende 188 des Beleuchtungsstabs 180 derart abgewinkelt, sodass das distale Ende 188 in der Bildaufnahmelage bündig mit dem Kameramodul 50 abschließt. In anderen Worten ist das distale Ende 188 des Beleuchtungsstabs 180 derart abgewinkelt, sodass das distale Ende 188 senkrecht und/oder ungefähr senkrecht zur Blickrichtung des Kameramoduls 50 in der Bildaufnahmelage verläuft.

Figur 7 zeigt eine distale Ansicht der beispielhaften Ausführungsform, die in Figur 6 dargestellt ist bzw. das distale Ende 188 des Beleuchtungsstab 180 mit der lichtauskoppelnde Fläche 140.

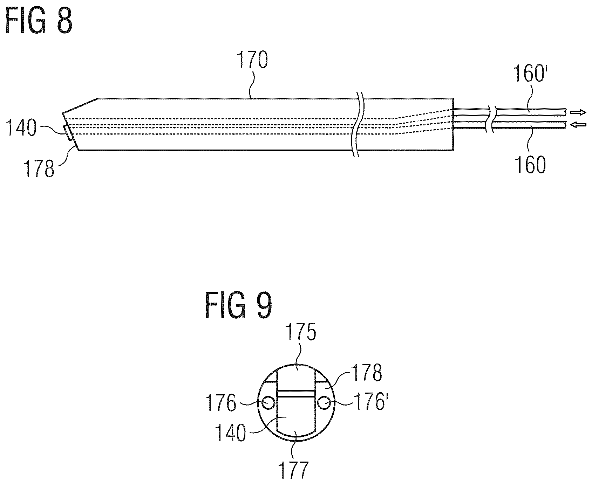

Figur 8 zeigt eine beispielhafte Ausführungsform des erfindungsgemäßen Aufnahmeschafts 170, in den der Beleuchtungsstab 180 aufgenommen ist. Die lichtauskoppelnde Fläche 140 des Beleuchtungsstab 180 tritt aus dem Aufnahmeschaft 170 hervor. Ferner weist der Aufnahmeschaft 170 eine Fluidleitung 160 und eine Fluidleitung 160' auf, die durch den Aufnahmeschaft 170 hindurchgeführt sind. Beispielsweise kann durch die Fluidleitung 160 ein Fluid, insbesondere Luft und/oder Wasser und/oder Sterilgas zu dem distalen Ende 178 des Aufnahmeschaft 170 geführt werden. Zudem kann beispielsweise durch die Fluidleitung 160' ein weiteres Fluid, beispielsweise ein verunreinigtes Fluid, insbesondere Blut und/oder Luft und/oder Wasser und/oder Sterilgas, von dem distalen Ende 178 des Aufnahmeschaft 170 abgesaugt werden.

Figur 9 zeigt eine distale Ansicht einer beispielhaften Ausführungsform, die in Figur 8 dargestellt ist bzw. das distale Ende 178 des Aufnahmeschafts 170. Das distale Ende 178 des Aufnahmeschafts 170 weist eine Öffnung 176 für die Fluidleitung 160 und eine Öffnung 176' für die Fluidleitung 160' auf. Zusätzlich kann das distale Ende 178 des Aufnahmeschafts 170 eine Öffnung 177 für die lichtauskoppelnde Fläche 140 aufweisen. Beispielsweise können die Öffnung 176 der Fluidleitung 160 und die Öffnung 176' der Fluidleitung 160' auf gegenüberliegenden Seiten der Öffnung 177 für die lichtauskoppelnde Fläche 140 angeordnet sein. Insbesondere können die Öffnung 176 der Fluidleitung 160 und die Öffnung 176' der Fluidleitung 160' auf gleicher Höhe auf gegenüberliegenden Seiten der Öffnung 177 für die lichtauskoppelnde Fläche 140 angeordnet sein. Darüber hinaus kann das distale Ende 178 des Aufnahmeschafts 170 die Aufnahme 175 aufweisen, in die das Kameramodul 50 aufgenommen werden kann.

### Bezugszeichenliste

- 10: Endoskopvorrichtung
- 20: Schaft
- 30: Schaftlängsachse
- 40: Betätigungsschaft
- 45: Längsachse
- 50: Kameramodul
- 60: Bilderfassungseinheit
- 61: erste Bilderfassungseinrichtung
- 62: zweite Bilderfassungseinrichtung
- 70: Kabel
- 80: distales Ende des Betätigungsschafts
- 90: distales Ende des Kameramoduls
- 100: Schwenkvorrichtung
- 110: erste Drehgelenk
- 120: zweites Drehgelenk
- 140: lichtauskoppelnde Fläche
- 150: Lichtleiter
- 160: Fluidleitung
- 170: Aufnahmeschaft
- 175: Aufnahme
- 176: Öffnung für die Fluidleitung
- 177: Öffnung für die lichtauskoppelnde Fläche
- 178: distales Ende des Aufnahmeschafts
- 180: Beleuchtungsstab
- 188: distales Ende des Beleuchtungsstabs
- 200: Trokar
- 210, 210', 210", 210‴: Führung

## Patentansprüche

1. Endoskopvorrichtung (10), umfassend:
einen Schaft (20) mit einer Schaftlängsachse (30);
einen Betätigungsschaft (40), der parallel zur Schaftlängsachse (30) bewegbar ist; und
ein Kameramodul (50), das schwenkbar an dem Schaft (20) gelagert ist und eine stereoskopische Bilderfassungseinheit (60) aufweist,
wobei das Kameramodul (50) durch ein Verschieben des Betätigungsschafts (40) relativ zum Kameramodul (50) und/oder durch ein Verschieben des Kameramoduls (50) relativ zum Betätigungsschaft (40), parallel zu der Schaftlängsachse (30), von einer Einführlage in eine Bildaufnahmelage auslenkbar ist,
wobei in der Bildaufnahmelage das Kameramodul (50) eine Blickrichtung definiert, die zu der Schaftlängsachse (30) abgewinkelt ist, und
wobei in der Bildaufnahmelage die stereoskopische Bilderfassungseinheit (60) zu einer Bildaufrichtung relativ zu dem Betätigungsschaft (40) drehbar gelagert ist.

2. Endoskopvorrichtung (10) nach Anspruch 1,
wobei die Bilderfassungseinheit (60) auch in der Einführlage des Kameramoduls (50) drehbar gelagert ist.

3. Endoskopvorrichtung (10) nach Anspruch 1 oder 2,
wobei die Bilderfassungseinheit (60) innerhalb des Kameramoduls (50) drehbar gelagert ist.

4. Endoskopvorrichtung (10) nach einem der vorherigen Ansprüche,
wobei die Bilderfassungseinheit (60) zur Bildaufrichtung mittels eines Kabels (70), insbesondere eines Torsionskabels und/oder eines Versorgungskabels, drehbar ist.

5. Endoskopvorrichtung (10) nach einem der vorherigen Ansprüche,
wobei in der Bildaufnahmelage der Betätigungsschaft (40) das Kameramodul (50) zumindest teilweise aufnimmt.

6. Endoskopvorrichtung (10) nach einem der vorherigen Ansprüche,
wobei in der Bildaufnahmelage eine Flächennormale einer distalen Endfläche des Betätigungsschafts (40) zu der Schaftlängsachse (30) abgewinkelt ist.

7. Endoskopvorrichtung (10) nach einem der vorherigen Ansprüche,
wobei in der Bildaufnahmelage ein distales Ende (80) des Betätigungsschafts (40) bündig mit einem distalen Ende des Kameramoduls (90) abschließt.

8. Endoskopvorrichtung (10) nach einem der vorherigen Ansprüche,
wobei in der Bildaufnahmelage das Kameramodul (50) auf dem Betätigungsschaft (40) aufliegt und insbesondere das Kameramodul (50) gegen den Betätigungsschaft (40) vorgespannt ist.

9. Endoskopvorrichtung (10) nach einem der vorherigen Ansprüche,
wobei das Kameramodul (50) durch eine Schwenkvorrichtung (100) an dem Schaft (20) schwenkbar gelagert ist.

10. Endoskopvorrichtung (10) nach Anspruch 9,
wobei das Kameramodul (50) durch die Schwenkvorrichtung (100) gegen den Betätigungsschaft (40) gedrückt wird.

11. Endoskopvorrichtung (10) nach einem der vorhergehenden Ansprüche,
wobei der Betätigungsschaft (40) zumindest eine lichtauskoppelnde Fläche (140), insbesondere lichtauskoppelnde Beleuchtungsfasern aufweist, die dazu eingerichtet ist, Beleuchtungslicht nach distal auszukoppeln.

12. Endoskopvorrichtung (10) nach einem der vorherigen Ansprüche,
wobei der Betätigungsschaft (40) zumindest einen Lichtleiter (150) und/oder zumindest eine Fluidleitung (160) umfasst, die sich entlang einer Längsachse (45) des Betätigungsschafts (40) erstrecken.

13. Endoskopvorrichtung (10) nach einem der vorherigen Ansprüche,
wobei zumindest eine Komponente des Betätigungsschafts (40) und/oder der Betätigungsschaft (40) als Einmalteil ausgebildet ist.

14. Endoskopvorrichtung (10) nach einem der vorherigen Ansprüche,
wobei der Betätigungsschaft (40) mittels eines Dichtelements zu dem Schaft (20) abdichtet.

15. Endoskopvorrichtung (10) nach einem der vorherigen Ansprüche,
wobei der Betätigungsschaft (40) einen Aufnahmeschaft (170) umfasst, wobei der Aufnahmeschaft (170) einen Kanal aufweist, in den der Schaft (20) einschiebbar ist.

16. Endoskopvorrichtung (10) nach einem der vorherigen Ansprüche,
wobei der Betätigungsschaft (40) einen Beleuchtungsstab (180) umfasst, der zumindest eine lichtauskoppelnde Fläche (140), insbesondere lichtauskoppelnde Beleuchtungsfasern, aufweist, die dazu eingerichtet ist, Beleuchtungslicht nach distal auszukoppeln.

17. Endoskopvorrichtung (10) nach einem der Ansprüche 15 oder 16,
wobei der Aufnahmeschaft (170) einen Kanal aufweist, in den der Beleuchtungsstab (180) einschiebbar ist.

18. Endoskopvorrichtung (10) nach einem der Ansprüche 15 bis 17,
wobei der Aufnahmeschaft (170) als Einmalteil ausgebildet ist; und/oder
wobei der Beleuchtungsstab (180) mehrfach verwendbar und insbesondere autoklavierbar ist.

19. Endoskopvorrichtung (10) nach einem der vorherigen Ansprüche,
wobei der Betätigungsschaft (40) und/oder der Aufnahmeschaft (170) eine Aufnahme (175) aufweist, in die das Kameramodul (50) zumindest teilweise aufgenommen ist, wobei das Kameramodul (50) bezüglich der Aufnahme (175) drehbar gelagert ist.

20. Endoskopvorrichtung (10) nach Anspruch 19,
wobei die Aufnahme (175) zu einer Längsachse (45) des Betätigungsschafts (40) und/oder zu einer Längsachse des Aufnahmeschafts (170) abgewinkelt ist.

21. Endoskopvorrichtung (10) nach einem der Ansprüche 19 oder 20,
wobei die Aufnahme (175) als eine halbrunde und/oder eckige Vertiefung ausgebildet ist, oder
die Aufnahme (175) an eine Form des Kameramoduls (50) angepasst ist.

22. System, umfassend:
eine Endoskopvorrichtung (10) nach einem der vorhergehenden Ansprüche; und
einen weiteren Betätigungsschaft, der anstelle des Betätigungsschafts (40) verwendbar ist,
wobei der Betätigungsschaft (40) eine erste Bildaufnahmelage mit einer ersten Blickrichtung definiert und wobei der weitere Betätigungsschaft eine zweite Bildaufnahmelage mit einer von der ersten Blickrichtung verschiedenen zweiten Blickrichtung definiert.
